# FASCICULE DE BREVET EUROPEEN

(11) **EP 1 233 953 B1**
(45) Date de publication et mention de la délivrance du brevet: **14.05.2003**
(21) Numéro de dépôt: 00981466.6
(22) Date de dépôt: 28.11.2000
(51) Int. Cl.: C07D 285/24, A61K 31/549, A61P 25/18

(54) **NOUVEAUX DERIVES DE BENZOTHIADIAZINES, LEUR PROCEDE DE PREPARATION ET LES COMPOSITIONS PHARMACEUTIQUES QUI LES CONTIENNENT**
BENZOTHIADIAZINDERIVATE, VERFAHREN ZU DEREN HERSTELLUNG UND SIE ENTHALTENDE PHARMAZEUTISCHE ZUSAMMENSETZUNGEN
NOVEL BENZOTHIADIAZINE DERIVATIVES, PREPARATION METHOD AND PHARMACEUTICAL COMPOSITIONS CONTAINING SAME

(30) Priorité: 30.11.1999 FR 9915034
(43) Date de publication de la demande: 28.08.2002
(73) Titulaire: Les Laboratoires Servier, 92415 Courbevoie Cedex (FR)
(72) Inventeur: PIROTTE, Bernard, B-4680 Oupeye (BE); DE TULLIO, Pascal, B-4020 Jupille sur Liège (BE); BOVERIE, Stéphane, B-4460 Bierset (BE); KEMPEN, Isabelle, B-4860 Pepinster (BE); LESTAGE, Pierre, F-78170 La Celle Saint Cloud (FR)
(86) Numéro de dépôt international: FR0003313
(87) Numéro de publication internationale: WO01040210

(56) Documents cités:
- WO-A-93/21170
- WO-A-93/21171
- WO-A-95/07899
- WO-A-98/12185
- CHEMICAL ABSTRACTS, vol. 77, no. 25, 18 décembre 1972 (1972-12-18) Columbus, Ohio, US; abstract no. 164185, GHELARDONI M.: "Hydrogenolysis of 3,4-dihydro-1,2,4-benzothiadiazine 1,1-dioxides" XP002145407 & ANN. CHIM. (ROME), vol. 62, no. 5, 1972, pages 373-384,

## Description

La présente invention concerne de nouveaux dérivés de benzothiadiazines, leur procédé de préparation, les compositions pharmaceutiques qui les contiennent ainsi que leur utilisation en tant que modulateurs des récepteurs AMPA.

Les substances activatrices des récepteurs AMPA, en facilitant la transmission synaptique glutamatergique dans le cerveau, ont un potentiel thérapeutique dans le traitement de la schizophrénie (brevet WO 97/07799) et des troubles cognitifs liés au vieillissement cérébral, notamment dans la maladie d'Alzheimer (Ito et coll., J. Physiol. 1990, 424, 543-553).

Un certain nombre de substances (aniracétam, cyclothiazide, diazoxide) sont aujourd'hui utilisées pour leurs propriétés activatrices des récepteurs AMPA.

Des dérivés de benzothiadiazines sont également décrits en tant que modulateurs allostériques positifs des récepteurs AMPA (brevet WO 98/12185 voir aussi WO 93/21170).

Il était donc particulièrement intéressant de synthétiser de nouveaux composés modulateurs des récepteurs AMPA afin d'augmenter la puissance, la sélectivité et la biodisponibilité des composés déjà décrits dans la littérature.

Plus spécifiquement, la présente invention concerne les composés de formule (I): 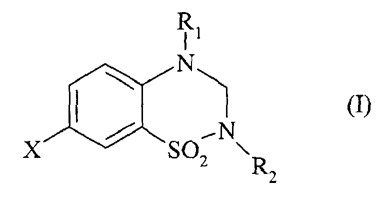 dans laquelle :
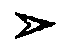 X représente un atome de fluor, de brome ou d'iode ou un groupement méthyle,
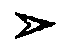 R₁ et R₂, identiques ou différents, représentent chacun un atome d'hydrogène ou un groupement alkyle (C₁-C₆) linéaire ou ramifié,
ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique, camphorique.

L'invention s'étend également au procédé de préparation des composés de formule (I) caractérisé en ce que l'on met en réaction un composé de formule (II) : 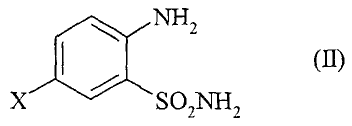 dans laquelle X a la même signification que dans la formule (I), avec l'orthoformiate d'éthyle,
pour conduire au composé de formule (III) : 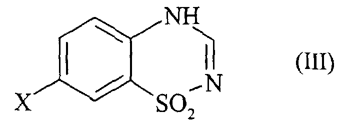 dans laquelle X a la même signification que précédemment,
que l'on met en réaction :
- soit avec un agent réducteur, pour conduire au composé de formule (Ia), cas particulier des composés de formule (I) : 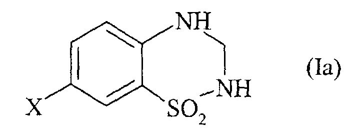 dans laquelle X a la même signification que précédemment,
que l'on met en réaction, lorsqu'on le souhaite, avec le dicarbonate de di(tert-butyle), pour conduire au composé de formule (IV): 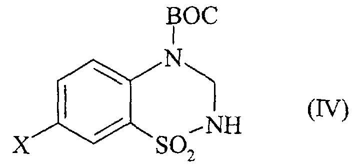 dans laquelle X a la même signification que précédemment et BOC représente le groupement tert-butoxycarbonyle,
que l'on met ensuite en réaction avec un composé de formule (V) :

R'₂-Y₂ (V)

dans laquelle R'₂ représente un groupement alkyle (C₁-C₆) linéaire ou ramifié, et Y₂ représente un groupe partant tel qu'un atome d'halogène ou un groupement tosylate, mésylate ou trifluorométhanesulfonate,
pour conduire après déprotection au composé de formule (Ib), cas particulier des composés de formule (I) : 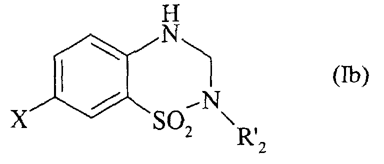 dans laquelle X et R'₂ ont la même signification que précédemment,
- soit avec un composé de formule (VI) :

   R'₁-Y₁ (VI)

   dans laquelle R'₁ représente un groupement alkyle (C₁-C₆) linéaire ou ramifié, et Y₁ représente un groupe partant tel qu'un atome d'halogène ou un groupement tosylate, mésylate ou trifluorométhanesulfonate,
pour conduire après réduction au composé de formule (Ic), cas particulier des composés de formule (I) : 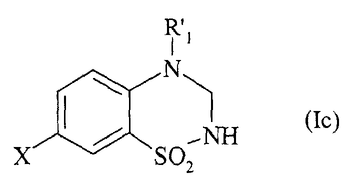 dans laquelle X et R'₁ ont la même signification que précédemment,
que l'on met en réaction, lorsqu'on le souhaite, avec le composé de formule (V) pour conduire au composé de formule (Id), cas particulier des composés de formule (I) : 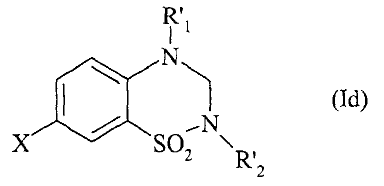 dans laquelle X, R'₁ et R'₂ ont la même signification que précédemment,
composés de formule (la), (Ib), (Ic) ou (Id) qui constituent l'ensemble des composés de formule (I), que l'on purifie, le cas échéant, selon une technique classique de purification, et que l'on transforme, si on le souhaite, en leurs sels d'addition à un acide pharmaceutiquement acceptable.

Le composé de formule (II) est obtenu selon le procédé décrit par Girard et coll. (J. Chem. Soc. Perkin I 1979, 1043-1047).

Les composés de la présente invention, outre le fait qu'ils soient nouveaux, présentent des propriétés activatrices des récepteurs AMPA qui les rendent utiles dans le traitement des déficits cognitifs associés au vieillissement cérébral et aux pathologies neurodégénératives telles que la maladie d'Alzheimer, la maladie de Parkinson, la maladie de Pick, la maladie de Korsakoff, les démences frontales et sous-corticales, ainsi que dans le traitement de la schizophrénie.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéfiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention et ne la limitent en aucune façon.

Les produits de départ utilisés sont des produits connus ou préparés selon des modes préparatoires connus.

Les structures des composés décrits dans les exemples ont été déterminées selon les techniques spectrophotométriques usuelles (infrarouge, RMN, spectrométrie de masse).

### EXEMPLE 1 : 7-Fluoro-2,3-dihydro-4H-1,2,4-benzothiadiazine 1,1-dioxyde

### Stade A : 2-Amino-5-fluorobenzènesulfonamide

Le produit attendu est obtenu selon le procédé décrit dans J. Chem. Soc. Perkin I 1979, 1043-1047 à partir de 4-fluoroaniline.

### Stade B : 7-Fluoro-4H-1,2,4-benzothiadiazine 1,1-dioxyde

A 10 mmoles du composé obtenu dans le stade précédent sont ajoutés 15 ml d'orthoformiate d'éthyle, puis le mélange est porté à reflux pendant 2 heures. Après retour à température ambiante, le précipité obtenu est filtré puis lavé et séché pour conduire au produit attendu.
*Point de fusion : 260-263°C*
*IR (KBr) : 3297, 3080, 1607, 1589, 1524, 1492, 1376, 1296, 1219, 1152 et 1134 cm*^{*-1*}

### Stade C : 7-Fluoro-2,3-dihydro-4H-1,2,4-benzothiadiazine 1,1-dioxyde

A 10 mmoles du composé obtenu dans le stade précédent en suspension dans l'eau sont ajoutées au goutte à goutte 10 mmoles de soude aqueuse à 5 % m/v, puis 50 mmoles de borohydrure de sodium en solution aqueuse à 5 % m/v. Après 1 heure d'agitation, le pH de la solution est ajusté à 6-7 par ajout d'une solution d'acide chlorhydrique 2N. Le précipité obtenu est filtré, lavé puis séché et recristallisé pour conduire au produit attendu.
*Point de fusion : 159-165°C*
*IR (KBr) : 3372, 3292, 1623, 1504, 1396, 1365, 1316, 1253, 1196 et 1158 cm*^{*-1*}

### EXEMPLE 2 : 7-Bromo-2,3-dihydro-4H-1,2,4-benzothiadiazine 1,1-dioxyde

### Stade A : 7-Bromo-4H-1,2,4-benzothiadiazine 1,1-dioxyde

Le produit attendu est obtenu selon le procédé décrit dans le stade B de l'Exemple 1 à partir de 2-amino-5-bromobenzènesulfonamide (décrit dans Il Farmaco 1974, 29, 47-57).
*Point de fusion : 279-281°C*
*IR (KBr) : 3233, 3163, 3082, 3026, 1615, 1577, 1523, 1478, 1376, 1288*, *1161, 1142 et 1090 cm*^{*-1*}

### Stade B : 7-Bromo-2,3-dihydro-4H-1,2,4-benzothiadiazine 1,1-dioxyde

Le produit attendu est obtenu selon le procédé décrit dans le stade C de l'Exemple 1 à partir du composé obtenu dans le stade précédent.
*Point de fusion : 207-212°C*
*IR (KBr) : 3421, 3282, 1596, 1498, 1386, 1360, 1319, 1310 et 1155 cm*^{*-1*}

### EXEMPLE 3 : 7-Bromo-4-éthyl-2,3-dihydro-4H-1,2,4-benzothiadiazine 1,1-dioxyde

### Stade A : 7-Bromo-4-éthyl-4H-1,2,4-benzothiadiazine 1,1-dioxyde

A 10 mmoles du composé obtenu dans le stade A de l'Exemple 2 en suspension dans l'acétonitrile sont ajoutées 30 mmoles de bromure d'éthyle et 30 mmoles de carbonate de potassium anhydre, puis le mélange est porté à reflux pendant 2 heures. Le solvant est ensuite évaporé et le résidu solide obtenu est dispersé dans l'eau. Le pH de la suspension est immédiatement ajusté à 7 à l'aide d'une solution d'acide chlorhydrique 2N. L'insoluble est filtré, lavé puis séché pour conduire au produit attendu.
*Point de fusion : 269-273°C*
*IR (KBr) : 1611, 1584, 1549*, *1473, 1434, 1410, 1395, 1305, 1279, 1258, 1162, 1120 et 1102 cm*^{*-1*}

### Stade B : 7-Bromo-4-éthyl-2,3-dihydro-4H-1,2,4-benzothiadiazine 1,1-dioxyde

A 10 mmoles du composé obtenu dans le stade précédent en suspension dans l'isopropanol sont ajoutées, par petites fractions, 30 mmoles de borohydrure de sodium finement dispersé. Après 40 minutes d'agitation à température ambiante, le solvant est évaporé et le résidu solide obtenu est dispersé dans l'eau. Le pH de la suspension est ensuite ajusté à 7, puis le solide est filtré, lavé, séché puis recristallisé pour conduire au produit attendu.
*Point de fusion : 122-124°C*
*IR (KBr) : 3222, 2975, 2964, 1594, 1547, 1497, 1470, 1336, 1320, 1263, 1167, 1153 et 1076 cm*^{*-1*}

### EXEMPLE 4 : 4-Ethyl-7-iodo-2,3-dihydro-4H-1,2,4-benzothiadiazine 1,1-dioxyde

Le produit attendu est obtenu selon le procédé décrit dans l'Exemple 3 à partir de 7-iodo-4*H*-1,2,4-benzothiadiazine 1,1-dioxyde (décrit dans Il Farmaco 1974, 29, 47-57).
*Point de fusion : 127-128°C*
*IR (KBr)* : *3224, 2972, 1588, 1495, 1335, 1319, 1268, 1186, 1153 et 1070 cm*^{*-1*}

### EXEMPLE 5 : 4-Ethyl-7-iodo-2-méthyl-2,3-dihydro-4H-1,2,4-benzothiadiazine 1,1-dioxyde

Le produit attendu est obtenu selon le procédé décrit dans le stade A de l'Exemple 3 à partir du composé décrit dans l'Exemple 4 et d'iodure de méthyle.
*Point de fusion : 139-141°C*
*IR (KBr) : 2972, 1589, 1492, 1458, 1412*, *1327, 1261, 1203, 1165, 1154 et 1104 cm*^{*-1*}

### EXEMPLE 6 : 4,7-Diméthyl-2,3-dihydro-4H-1,2,4-benzothiadiazine 1,1-dioxyde

### Stade A : 7-Méthyl-4H-1,2,4-benzothiadiazine-1,1-dioxyde

Le produit attendu est obtenu selon le procédé décrit dans le stade B de l'Exemple 1 à partir de 2-amino-5-méthylbenzènesulfonamide (décrit dans J. Chem. Soc. Perkin I 1979, 1043-1047).
*Point de fusion : 298-299°C*
*IR (KBr) : 3272, 3105, 3042, 1605, 1590, 1522, 1498, 1379, 1286, 1155, 1140 et 1067 cm*^{*-1*}

### Stade B : 4,7-Diméthyl-2,3-dihydro-4H-1,2,4-benzothiadiazine 1,1-dioxyde

Le produit attendu est obtenu selon le procédé décrit dans l'Exemple 3 à partir du composé décrit dans le stade précédent et d'iodure de méthyle.
*Point de fusion : 149-151°C*
*IR (KBr) : 3228, 1619, 1515, 1318, 1297, 1205, 1163, 1148 et 1071 cm*^{*-1*}

### EXEMPLE 7 : 4-Ethyl-7-méthyl-2,3-dihydro-4H-1,2,4-benzothiadiazine 1,1-dioxyde

Le produit attendu est obtenu selon le procédé décrit dans l'Exemple 3 à partir du composé décrit dans le stade A de l'Exemple 6 et de bromure d'éthyle.
*Point de fusion : 110-111°C*
*IR (KBr) : 3235, 2974, 1621, 1512, 1338, 1316, 1299, 1266, 1251, 1188 et 1152 cm-1*

### EXEMPLE 8 : Etude des courants excitateurs induits par l'AMPA dans les oocytes de Xenopus

Des ARNm sont préparés à partir de cortex cérébral de rat mâle Wistar par la méthode au guanidium thiocyanate/phénol/chloroforme. Les ARNm Ploy (A+) sont isolés par chromatographie sur oligo-dT cellulose et injectés à raison de 50 ng par oocyte. Les oocytes sont laissés 2 à 3 jours en incubation à 10°C pour permettre l'expression des récepteurs puis stockés à 8-10°C.

L'enregistrement électrophysiologique est réalisé dans une chambre en plexiglass à 20-24°C en milieu OR2 (J. Exp. Zool., 1973, 184, 321-334) par la méthode du « voltage-clamp » à 2 électrodes, une 3^{ème} électrode placée dans le bain servant de référence.

Tous les composés sont appliqués *via* le milieu d'incubation et le courant électrique est mesuré à la fin de la période d'application. L'AMPA est utilisé à la concentration de 30 µM. Pour chaque composé étudié, on définit la concentration doublant (EC2X) ou quintuplant (EC5X) l'intensité du courant induit par l'AMPA seul (50 à 100 nA).

Les composés de l'invention potentialisent fortement les effets excitateurs de l'AMPA. A titre d'exemple, les composés des Exemples 3 et 4 présentent les EC2X et EC5X mcntionnécs dans le tableau ci-dessous :

| *Composé* | *EC2X (µM)* | *EC5X (µM)* |
|---|---|---|
| Exemple 3 | 29 ± 6 | 78 ± 16 |
| Exemple 4 | 95 ± 31 | >300 |

### EXEMPLE 9 : Reconnaissance sociale chez le rat Wistar

Initialement décrit en 1982 par THOR et HOLLOWAY (J. Comp. Physiol., 1982, 96, 1000-1006), le test de la reconnaissance sociale a été ensuite proposé par différents auteurs (DANTZER et coll., Psychopharmacology, 1987, 91, 363-368; PERIO et coll., Psychopharmacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester et, 2 heures plus tard, est remis en présence (5 minutes) du jeune rat. Le comportement de reconnaissance sociale est alors à nouveau observé et la durée en est mesurée. Le critère de jugement est la différence (T₂-T₁), exprimée en secondes, des temps de « reconnaissance » des 2 rencontres.

Les résultats obtenus montrent une différence (T₂-T₁) comprise entre (-15) et (-30) s pour des doses allant de 0,3 à 3 mg/kg. Ceci montre que les composés de l'invention augmcntcnt la mémorisation de façon très importante, et à faible dose.

### EXEMPLE 10 : Reconnaissance d'objet chez le rat Wistar

Le test de reconnaissance d'objet chez le rat Wistar a été initialement développé par ENNACEUR et DECACOUR (Behav. Brain Res., 1988, 31, 47-59). Ce test est basé sur l'activité exploratoire spontanée de l'animal et possède les caractéristiques de la mémoire épisodique chez l'homme. Sensible au vieillissement (SCALL et coll., Eur. J. Pharmacol., 1997, 325, 173-180), ainsi qu'aux dysfonctionnements cholinergiques (BARTOLINI et coll., Pharm. Biochem. Behav. 1996, 53(2), 277-283), ce test de mémoire est fondé sur l'exploration différentielle de 2 objets de forme assez similaire, l'un familier, l'autre nouveau. Préalablement au test, les animaux sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{ème} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement au véhicule soit par voie IP 30 minutes avant chaque session, soit par voie orale 60 minutes avant chaque session, explorent de façon identique l'objet familier et l'objet nouveau, ce qui signe l'oubli de l'objet déjà présenté. Les animaux traités par un composé facilitateur mnémocognitif, explorent de façon préférentielle l'objet nouveau, ce qui signe le souvenir de l'objet déjà présenté.

Les résultats obtenus montrent une différence Delta comprise entre 5 et 10s, pour des doses allant de 0,3 à 3 mg/kg par voie IP et allant de 3 à 30 mg/kg par voie orale, ce qui montre que les composés de l'invention augmentent la mémorisation de façon importante, et à faible dose.

### EXEMPLE 11 : Composition pharmaceutique

Formule de préparation pour 1000 comprimés dosés à 10 mg :
Composé de l'exemple 1 10 g
Hydroxypropylcellulose 2 g
Amidon de blé 10 g
Lactose 100 g
Stéarate de magnésium 3 g
Talc 3 g

## Revendications

1. Composé de formule (I) : 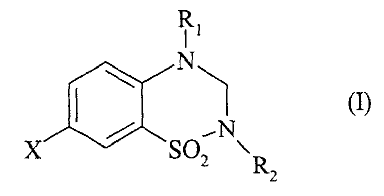 dans laquelle :
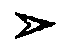 X représente un atome de fluor, de brome ou d'iode ou un groupement méthyle,
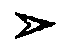 R₁ et R₂, identiques ou différents, représentent chacun un atome d'hydrogène ou un groupement alkyle (C₁-C₆) linéaire ou ramifié,
ainsi que leurs sels d'addition à un acide pharmaceutiquement acceptable.

2. Composé de formule (I) selon la revendication 1 qui est le 7-bromo-4-éthyl-2,3-dihydro-4*H*-1,2,4-benzothiadiazine 1,1-dioxyde.

3. Composé de formule (I) selon la revendication 1 qui est le 4-éthyl-7-iodo-2,3-dihydro-4*H*-1,2,4-benzothiadiazine 1,1-dioxyde.

4. Procédé de préparation des composés de formule (I) **caractérisé en ce que** l'on met en réaction : 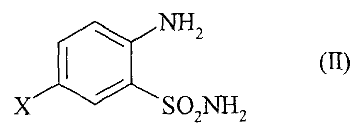 dans laquelle X a la même signification que dans la formule (I), avec l'orthoformiate d'éthyle, pour conduire au composé de formule (III) : 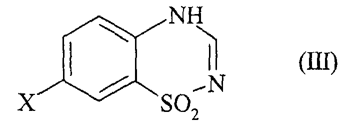 dans laquelle X a la même signification que précédemment,
que l'on met en réaction :
- soit avec un agent réducteur, pour conduire au composé de formule (Ia), cas particulier des composés de formule (I) : 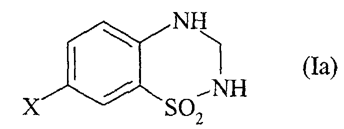 dans laquelle X a la même signification que précédemment,
que l'on met en réaction, lorsqu'on le souhaite, avec le dicarbonate de di(tert-butyle), pour conduire au composé de formule (IV) : 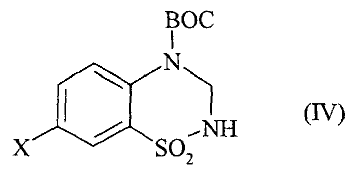 dans laquelle X a la même signification que précédemment et BOC représente le groupement tert-butoxycarbonyle,
que l'on met ensuite en réaction avec un composé de formule (V) :
R'₂-Y₂ (V)
dans laquelle R'₂ représente un groupement alkyle (C₁-C₆) linéaire ou ramifié, et Y₂ représente un groupe partant tel qu'un atome d'halogène ou un groupement tosylate, mésylate ou trifluorométhanesulfonate, pour conduire après déprotection au composé de formule (Ib), cas particulier des composés de formule (I) : 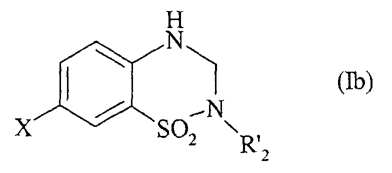 dans laquelle X et R'₂ ont la même signification que précédemment,
- soit avec un composé de formule (VI) :
R'₁-Y₁ (VI)
dans laquelle R'₁ représente un groupement alkyle (C₁-C₆) linéaire ou ramifié, et Y₁ représente un groupe partant tel qu'un atome d'halogène ou un groupement tosylate, mésylate ou trifluorométhanesulfonate,
pour conduire après réduction au composé de formule (Ic), cas particulier des composés de formule (I) : 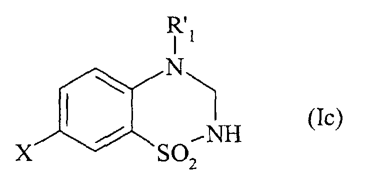 dans laquelle X et R'₁ ont la même signification que précédemment,
que l'on met en réaction, lorsqu'on le souhaite, avec le composé de formule (V) pour conduire au composé de formule (Id), cas particulier des composés de formule (I) : 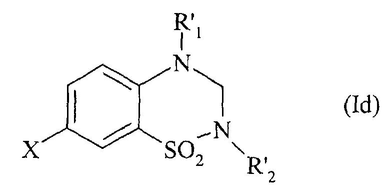 dans laquelle X, R'₁ et R'₂ ont la même signification que précédemment,
composés de formule (Ia), (Ib), (Ic) ou (Id) qui constituent l'ensemble des composés de formule (1), que l'on purifie, le cas échéant, selon une technique classique de purification, et que l'on transforme, si on le souhaite, en leurs sels d'addition à un acide pharmaceutiquement acceptable.

5. Compositions pharmaceutiques contenant comme principe actif un composé selon l'une quelconque des revendications 1 à 3, seul ou en combinaison avec un ou plusieurs véhicules inertes, non toxiques et pharmaceutiquement acceptables.

6. Compositions pharmaceutiques selon la revendication 5 utiles en tant que médicaments comme modulateurs AMPA.

7. Compositions pharmaceutiques selon la revendication 6 utiles en tant que médicaments comme facilitateurs mnémocognitifs.

8. Compositions pharmaceutiques selon la revendication 6 utiles en tant que médicaments dans le traitement de la schizophrénie.

## Claims

1. Compound of formula (I) : 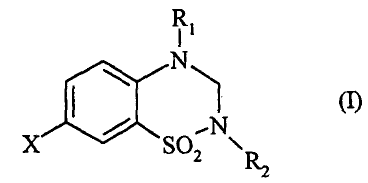 wherein:
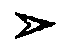 X represents a fluorine, bromine or iodine atom or a methyl group,
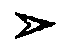 each of R₁ and R₂, which may be identical or different, represents a hydrogen atom or a linear or branched (C₁-C₆)alkyl group,
and addition salts thereof with a pharmaceutically acceptable acid.

2. Compound of formula (I) according to claim 1 which is 7-bromo-4-ethyl-2,3-dihydro-4*H*-1,2,4-benzothiadiazine 1,1 -dioxide.

3. Compound of formula (I) according to claim 1 which is 4-ethyl-7-iodo-2,3-dihydro-4*H*-1,2,4-benzothiadiazine 1,1-dioxide.

4. Process for the preparation of compounds of formula (I), **characterised in that**: 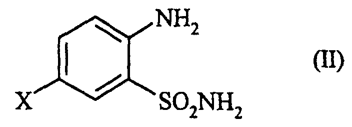 wherein X is as defined for formula (I), is reacted with ethyl orthoformate,
to yield a compound of formula (III) : 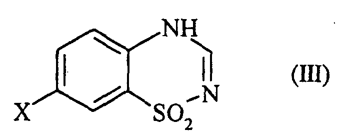 wherein X is as defined hereinbefore,
which is reacted:
- either with a reducing agent, to yield a compound of formula (Ia), a particular case of the compounds of formula (I) : 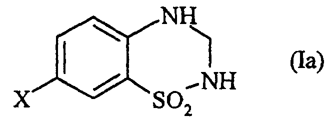 wherein X is as defined hereinbefore,
which is reacted, if desired, with di(tert-butyl) dicarbonate, to yield a compound of formula (IV): 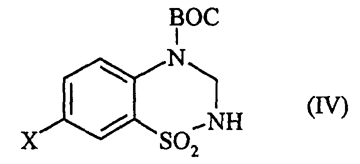 wherein X is as defined hereinbefore and BOC represents the group tert-butoxycarbonyl,
which is then reacted with a compound of formula (V):
R'₂-Y₂ (V)
wherein R'₂ represents a linear or branched (C₁-C₆)alkyl group, and Y₂ represents a leaving group, such as a halogen atom or a tosylate, mesylate or trifluoromethanesulphonate group,
to yield, after deprotection, a compound of formula (Ib), a particular case of the compounds of formula (I) : 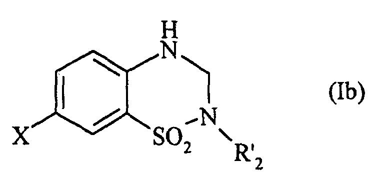 wherein X and R'₂ are as defined hereinbefore,
- or with a compound of formula (VI) :
R'₁-Y₁ (VI)
wherein R'₁ represents a linear or branched (C₁-C₆)alkyl group, and Y₁ represents a leaving group, such as a halogen atom or a tosylate, mesylate or trifluoromethanesulphonate group,
to yield, after reduction, a compound of formula (Ic), a particular case of the compounds of formula (I) : 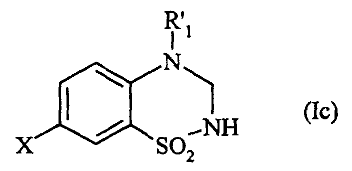 wherein X and R'₁ are as defined hereinbefore,
which is reacted, if desired, with a compound of formula (V) to yield a compound of formula (Id), a particular case of the compounds of formula (I) : 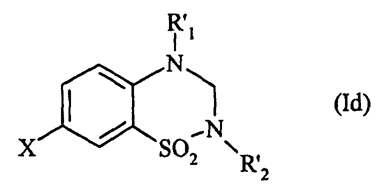 wherein X, R'₁ and R'₂ are as defined hereinbefore,
which compounds of formulae (Ia), (Ib), (Ic) and (Id), which constitute the totality of the compounds of formula (I), are purified, if necessary, according to a conventional purification technique, and converted, if desired, into addition salts with a pharmaceutically acceptable acid.

5. Pharmaceutical compositions comprising as active ingredient a compound according to any one of claims 1 to 3, alone or in combination with one or more inert, non-toxic pharmaceutically acceptable carriers.

6. Pharmaceutical compositions according to claim 5 for use as medicaments as AMPA modulators.

7. Pharmaceutical compositions according to claim 6 for use as medicaments as mnemocognitive facilitators.

8. Pharmaceutical compositions according to claim 6 for use as medicaments in the treatment of schizophrenia.

## Patentansprüche

1. Verbindung der Formel (I): 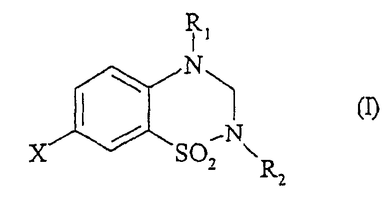 in der:
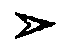 X ein Fluoratom, Bromatom, lodatom oder eine Methylgruppe, und
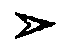 R₁ und R₂, die gleichartig oder verschieden sind, jeweils ein Wasserstoffatom oder eine geradkettige oder verzweigte (C₁-C₆)-Alkylgruppe bedeuten.
sowie deren Additionssalze mit einer pharmazeutisch annehmbaren Säure.

2. Verbindung der Formel (I) nach Anspruch 1, nämlich 7-Brom-4-ethyl-2,3-dihydro-4*H*-1,2,4-benzothiadiazin-1,1-dioxid.

3. Verbindung der Formel (I) nach Anspruch 1, nämlich 4-Ethyl-7-iod-2,3-dihydro-4*H*-1,2,4-benzothiadiazin-1,1-dioxid.

4. Verfahren zur Herstellung der Verbindungen der Formel (I), **dadurch gekennzeichnet, daß** man: 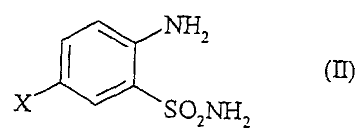 in der X die bezüglich der Formel (I) angegebenen Bedeutungen besitzt, mit Orthoameisensäureethylester umsetzt,
zur Bildung der Verbindung der Formel (III): 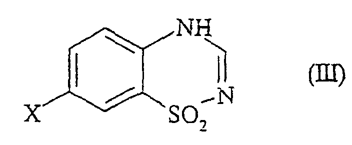 in der X die oben angegebenen Bedeutungen besitzt.
welche man:
entweder mit einem Reduktionsmittel umsetzt zur Bildung der Verbindung der Formel (Ia), einem Sonderfall der Verbindungen der Formel (I): 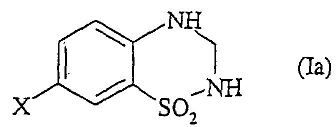 in der X die oben angegebenen Bedeutungen besitzt,
welche man gewünschtenfalls mit Di-(tert,-butyl)-dicarbonat umsetzt zur Bildung der Verbindung der Formel (IV): 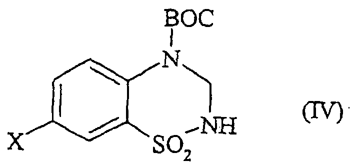 in der X die oben angegebenen Bedeutungen besitzt und BOC für eine tert.-Butoxycarbonylgruppe steht,
welche man anschließend mit einer Verbindung der Formel (V) umsetzt:
R'₂ - Y₂ (V)
in der R'₂ eine geradkettige oder verzweigte (C₁-C₆)-Alkylgruppe und Y₂ eine austretende Gruppe, wie ein Halogenatom oder eine Tosylat-, Mesylat- oder Trifluormethansulfonat-gruppe bedeuten,
so daß man nach der Abspaltung der Schutzgruppe die Verbindung der Formel (Ib) erhält, einem Sonderfall der Verbindungen der Formel (I): 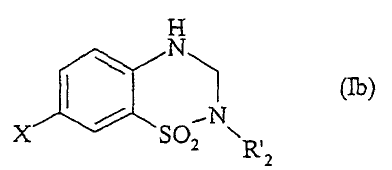 in der X und R'₂ die oben angegebenen Bedeutungen besitzen,
- oder mit einer Verbindung der Formel (VI) umsetzt:
R'₁-Y₁ (VI)
in der R'₁ eine geradkettige oder verzweigte (C₁-C₆)-Alkylgruppe und Y₁ eine austretende Gruppe, wie ein Halogenatom oder eine Tosylat-, Mesylat- oder Trifluormethansulfonat-gruppe bedeuten,
so daß man nach der Reduktion die Verbindung der Formel (Ic) erhält. einem Sonderfall der Verbindungen der Formel (I): 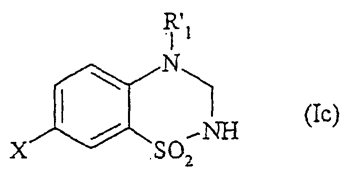 in der X und R'₁ die oben angegebenen Bedeutungen besitzen,
welche man gewünschtenfalls mit der Verbindung der Formel (V) umsetzt zur Bildung der Verbindung der Formel (Id), einem Sonderfall der Verbindungen der Formel (I): 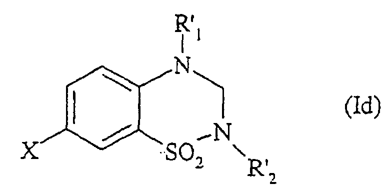 in der X, R'₁ und R'₂ die oben angegebenen Bedeutungen besitzen,
welche Verbindungen der Formeln (Ia), (Ib), (Ic) oder (Id), welche die Gesamtheit der Verbindungen der Formel (I) darstellen, man gegebenenfalls mit Hilfe einer klassischen Reinigungsmethode reinigt und gewünschtenfalls in ihre Additionssalze mit einer pharmazeutisch annehmbaren Säure umwandelt.

5. Pharmazeutische Zubereitungen enthaltend als Wirkstoff eine Verbindung nach irgendeinem der Ansprüche 1 bis 3 allein oder in Kombination mit einem oder mehreren inerten, nichttoxischen und pharmazeutisch annehmbaren Trägermaterialien.

6. Pharmazeutische Zubereitungen nach Anspruch 5, die als AMPA-modulierende Arzneimittel nützlich sind.

7. Pharmazeutische Zubereitungen nach Anspruch 6, die als Gedächtnis/Erkenntnis-erleichternde Arzneimittel nützlich sind.

8. Pharmazeutische Zubereitungen nach Anspruch 6, die als Arzneimittel bei der Behandlung der Schizophrenie nützlich sind.
